# FASCICULE DE BREVET EUROPEEN

(11) **EP 1 727 441 B1**
(45) Date de publication et mention de la délivrance du brevet: **29.10.2008**
(21) Numéro de dépôt: 05739432.2
(22) Date de dépôt: 11.03.2005
(51) Int. Cl.: A41C 3/00, A41C 3/10, A41D 13/05, A61F 5/02

(54) **TISSU DE CONTENTION A POCHES**
STÜTZGEWEBE MIT TASCHEN
RETAINING FABRIC HAVING POCKETS

(30) Priorité: 22.03.2004 FR 0450564
(43) Date de publication de la demande: 06.12.2006
(73) Titulaire: BERTHEAS & CIE, F-42400 Saint Chamond (FR)
(72) Inventeur: MOULIN, Hervé, F-42000 SAINT-ETIENNE (FR)
(74) Mandataire: Dupuis, François
(86) Numéro de dépôt international: PCT/FR2005/050156
(87) Numéro de publication internationale: WO 2005/092238

(56) Documents cités:
- DE-A1- 3 049 566
- DE-A1- 4 419 410
- FR-A- 2 787 124
- US-A- 2 957 475
- US-A- 5 070 866

## Description

L'invention se rattache au secteur technique de la confection d'orthèses, bandages, à usage médical, sportif, et de protection notamment.

La démarche inventive du demandeur a été initiée à partir d'un problème posé concernant les ceintures de soutien lombaire.

Les ceintures de ce type à base textile sont bien connues et sont agencées avec des moyens de rigidification du type baleines métalliques ou plaques rapportées sur le tissu de contention.

Une telle ceinture décrit le document US-A-2957475, par exemple.

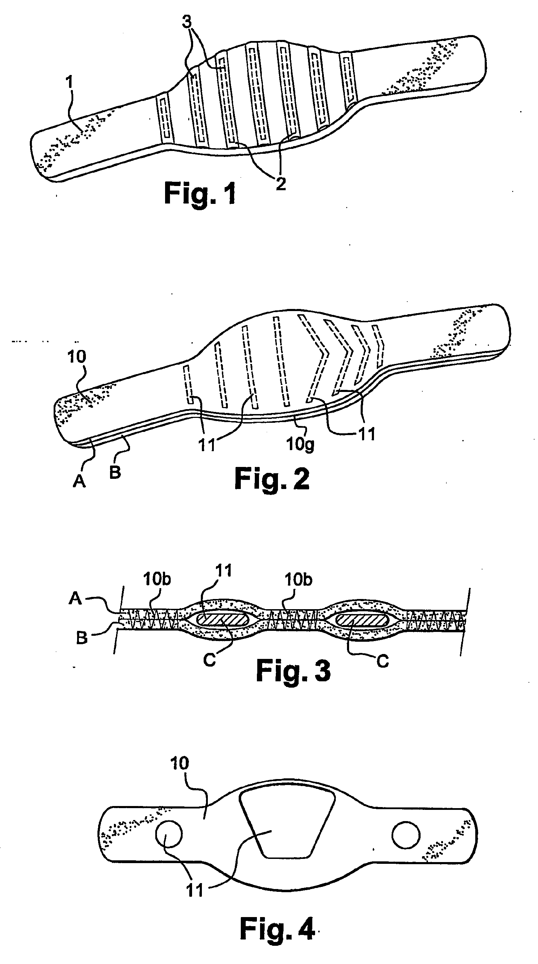

On a représenté, figure 1, une ceinture selon l'art antérieur. Celle-ci est référencée par (1) et comprend, dans sa partie médiane correspondant à l'appui dorsal sur l'individu, une pluralité de cache-baleines (2) qui ont été rapportés et sont cousus ou autrement solidarisés sur l'une des faces de la ceinture. Ces cache-baleines qui sont fermés, après placement des baleines (3), sont généralement réalisés en un matériau différent de celui constitutif de la ceinture, et sont par exemple dans des matériaux, tels que ruban tissé, simili cuir, ou certains plastiques. En pratique, ces cache-baleines sont obtenus à partir d'une bande plate qui, pendant la confection, est rapportée en applique sur la ceinture pour former des logements pour la réception des baleines. Ces cache-baleines, sous forme de bandes plates, sont ainsi rapportés et fixés par couture ou similaires sur le tissu de contention, la baleine étant prise en sandwich entre le corps du tissu et ladite bande. Les logements ainsi formés sont disposés de manière définitive, selon un écartement pré-établi. Selon la réalisation, les ceintures peuvent présenter des logements supplémentaires disposés dans la partie en prolongement de la partie d'application dorsale pour venir sur les flancs latéraux de l'individu porteur. Ainsi, les ceintures sont fabriquées et conçues d'une manière définitive selon des tailles pré-établies, et avec un nombre prédéfini dans une gamme en fonction de l'âge, de la taille et de la morphologie de l'individu porteur.

En pratique, ce type de ceinture, qui est largement exploité par de nombreux fabricants, présente de nombreux inconvénients ou limitations.

Un premier inconvénient réside tout d'abord dans les coûts de fabrication car il est nécessaire de fabriquer indépendamment la ceinture textile elle-même, puis les logements. On procède alors à l'introduction, des moyens de rigidification dans lesdits logements qui sont ensuite fermés puis, ou en même temps, solidarisés à la ceinture par couture ou autres moyens de liaison. En variante, selon les techniques de fabrication, les logements peuvent être présentés non fermés sur la ceinture aux endroits appropriés, puis un opérateur introduit les baleines une à une, et l'opération se poursuit par la fermeture de l'ensemble. Le temps d'intervention est donc élevé, les manipulations nombreuses. En outre, le problème de positionnement des logements sur la ceinture requiert un contrôle, un traçage et un mesurage parfait des distances afin de mettre en place une pluralité de logements récepteurs desdits moyens de rigidification.

Un autre inconvénient réside dans cette mise en oeuvre définitive des logements sur la ceinture de sorte que bien qu'il puisse y avoir plusieurs tailles de ceinture, il n'y a pas toujours adéquation parfaite entre la ceinture proposée au porteur et à la morphologie de celui-ci.

Un autre inconvénient réside dans le fait que le volume de la ceinture ainsi agencée est important car les logements récepteurs des baleines sont en saillie d'un côté, et l'enroulement de la ceinture pour rangement ou emballage requiert un volume important.

Un autre inconvénient qui découle de la technique de fabrication de ces ceintures, réside dans le fait que celles-ci ont une seule application définie et pré-établie.

Malgré ces inconvénients ou limitations, les ceintures sont ainsi et à défaut d'autres solutions et propositions mises en oeuvre selon cette conception.

La démarche du demandeur a donc été de réfléchir à un nouveau concept de tissu de contention autorisant lors de la fabrication de ceintures ou autres dispositifs tout d'abord une réduction des coûts de fabrication, mais permettant aussi d'envisager de plus larges possibilités d'agencement.

Par ailleurs, le demandeur a souhaité proposer un tissu de contention qui puisse facilement être personnalisé à la morphologie de l'utilisateur porteur et de ses besoins.

En d'autres termes, le demandeur a réfléchi à la conception d'un tissu de contention qui permette, par la mise en oeuvre d'une nouvelle technique de fabrication, l'introduction et la mise en place de moyens offrant un éventail d'applications beaucoup plus large à usage médical, sportif et de protection en outre de l'application initiale du type des ceintures de soutien lombaire.

Les recherches développées par le demandeur l'ont ainsi amené à concevoir un nouveau type de tissu de contention qui réponde à l'ensemble des problèmes posés avec une réduction substantielle des coûts de fabrication.

Ainsi, selon une première caractéristique de l'invention, le tissu de contention, du type agencé avec des logements de réception confectionnés et destinés à recevoir notamment des moyens de rigidification, est remarquable en ce qu'il comprend deux nappes tissées solidarisées par liage et s'écartant l'une de l'autre de manière non liée ponctuellement lors de leur fabrication pour définir directement une ou des poches profilées et agencées pour recevoir un ou des moyens introduits après la fabrication, et en ce que la ou lesdites poches étant donc incorporées dans le corps du tissu de contention, chaque poche présentant une ouverture d'introduction d'un des moyens.

Selon une autre caractéristique de l'invention, le ou lesdits moyens introduits dans le tissu de contention sont des raidisseurs du type baleines ou autres.

Selon une autre caractéristique de l'invention, le ou lesdits moyens introduits dans le tissu de contention ont une fonction thérapeutique et/ou de confort.

Selon une autre caractéristique de l'invention, le procédé de fabrication du tissu de contention est remarquable en ce qu'il consiste à réaliser deux nappes tissées qui sont superposées et solidarisées par des fils de liage pour constituer des parties unitaires du tissu de contention, puis, selon un cycle pré-établi, à séparer lesdites nappes selon une distance donnée, puis à les rapprocher et solidariser à nouveau pour constituer successivement une ou des poches profilées pour l'introduction et la réception ultérieure de moyens extérieurs, et en ce que, simultanément à la fabrication du tissu de contention, la ou lesdites poches sont fermées partiellement en laissant une ouverture d'introduction pour la mise en place du ou desdits moyens précités.

Selon une autre caractéristique du procédé de fabrication, les lisières sont établies, l'une des lisières étant fermée sur la totalité de la longueur du tissu de contention, et l'autre lisière étant fermée partiellement à l'exception de la zone des poches pour autoriser l'insertion desdits moyens dans lesdites poches.

Ces caractéristiques et d'autres encore ressortiront bien de la suite de la description.

Pour illustrer l'objet de l'invention d'une manière non limitative aux figures des dessins où :
- la figure 1 est une vue en perspective d'une ceinture de soutien lombaire, selon l'art antérieur,
- la figure 2 est une vue en perspective d'un tissu de contention, selon l'invention dans une première mise en oeuvre,
- la figure 3 est une vue partielle et à grande échelle en coupe illustrant le tissu de contention selon l'invention avec des poches disposées régulièrement pour la réception de moyens raidisseurs du type baleine,
- la figure 4 est une vue en plan à caractère schématique d'un tissu de contention, selon l'invention, agencé avec des poches établies dans la largeur du tissu pour la réception de moyens thérapeutiques et/ou de confort tels que des pelotes en silicone ou autres,
- les figures 5A, 5B, 5C sont des exemples non limitatifs illustrant la confection de poches sur un tissu de contention, selon différentes variantes de forme et d'espacement.

Afin de rendre plus concret l'objet de l'invention, on le décrit maintenant d'une manière non limitative illustrée aux dessins.

Le tissu de contention, selon l'invention, est référencé dans son ensemble par (10). Il est réalisé à partir de deux nappes tissées (A - B), superposées, qui ont toutes contextures et armures avec fils de chaîne, fils de trame connus dans toutes configurations possibles avec élasticité, sur tout ou partie de la longueur des nappes tissées.

Ces deux nappes tissées (A - B) sont, selon l'invention, solidarisées entre elles lors du tissage par des fils de liage (10b), fils totalement intégrés à l'ensemble des fils de chaîne travaillant selon un pas pré-établi en fonction de la ou des poches (C) à réaliser.

Ainsi, selon l'invention, le tissu de contention fabriqué forme, à certains endroits dans le corps du tissu, des poches profilées correspondant à des zones non liées des deux nappes tissées (A - B). En pratique, à l'endroit désiré de formation des poches précitées, les fils de liage (10b) sont déviés et acheminés différemment sur les dites nappes tissées, et cela selon un pas pré-établi correspondant aux dimensions et à la configuration de la ou des poches à obtenir, puis lesdits fils de liage sont à nouveau déviés pour réintégrer, après formation de la poché, leur trajectoire initiale dans le corps des deux nappes tissées afin d'assurer leur fonction de liage. Les poches (C) sont ainsi définies par l'espace ou volume entre les deux nappes tissées (A - B) qui peuvent s'écarter l'une de l'autre n'étant pas liées à ou aux endroits considérés. Les poches sont délimitées par le travail des fils de liage intégrés dans les deux nappes tissées immédiatement avant et après leur déviation ou trajectoire, comme précisé ci-avant.

Ainsi, et selon l'invention, on fabrique en continu, et en une seule fois, sur le métier à tisser, à l'aide de mécanismes connus, ledit tissu de contention selon l'invention. Le nombre de poches ainsi créé sur la longueur du tissu de contention dépendra de l'application souhaitée pour le tissu de contention, selon qu'il s'agit d'une ceinture de contention par exemple ou pour d'autres utilisations. Il sera par ailleurs facile de programmer l'automatisme du métier à tisser pour réaliser le tissu de contention avec le nombre de poches souhaitées, leur espacement respectif, leur largeur en fonction de la nature et des caractéristiques des moyens (11) introduits tels que baleines ou autres. Par ailleurs, la longueur des poches réceptrices peut être établie dans toute la largeur du tissu de contention, mais aussi partiellement. Il suffit pour cela d'adapter la trajectoire des fils de liage, selon la programmation choisie. Ainsi, selon l'invention, les moyens intégrés dans les poches qui peuvent avoir une fonction de raidisseurs sous forme de baleines sont totalement intégrés dans le corps du tissu de contention, en laissant apparentes les parties tissées des nappes tissées, conférant un agencement nouveau et un aspect extérieur spécifique au tissu de contention.

Par ailleurs, l'un des intérêts de l'invention est qu'il est possible de fabriquer un tissu de contention selon le procédé de l'invention, mais de ne placer qu'un nombre plus réduit de baleines ou autres moyens par rapport au nombre de poches, en permettant donc une adaptation de la confection de la ceinture ou autres, en fonction des besoins et/ou la morphologie du porteur. En d'autres termes, on peut faire varier le nombre de baleines ou autres et leur disposition dans le tissu de contention, car l'accès aux dites baleines ou autres moyens est possible par l'ouverture des poches correspondantes. Lesdites ouvertures de poches peuvent rester libres ou fermées par couture ou autrement.

Les lisières (10g) sont établies, l'une étant fermée sur toute la longueur du tissu de contention et l'autre lisière étant fermée partiellement à l'exception de la zone des poches pour autoriser l'insertion desdits moyens dans lesdites poches.

La mise en oeuvre de l'invention, par le procédé de fabrication précité, permet de constituer des poches dans le corps du tissu de contention pour disposer des moyens raidisseurs sous forme de baleines mais aussi des plaques, ainsi que des moyens ayant d'autres utilisations telles que pelotes, matériau thermoformé, poches de gel, poches de diffusion et autres. On a représenté figure 4, à titre d'exemple et de manière schématique, différentes configurations de poches qui peuvent être de configuration géométrique identique ou différente pour recevoir des moyens (11) identiques ou différents.

La configuration des poches peut être établie lors de la fabrication selon des formes non rectilignes, à chevrons, par exemple, mais aussi selon des formes géométriques correspondant à celles des moyens et composants à retenir, l'essentiel étant qu'une ouverture appropriée soit établie pour chaque poche pour l'insertion de ces dits moyens. A cet effet, lesdites ouvertures de poches peuvent déboucher près ou le long d'une lisière du tissu de contention, mais aussi dans la largeur du tissu de contention.
Ainsi, le procédé de l'invention offre de nombreuses possibilités d'applications à des fins médicales, sportives, de protection, thérapeutiques et/ou de confort notamment.

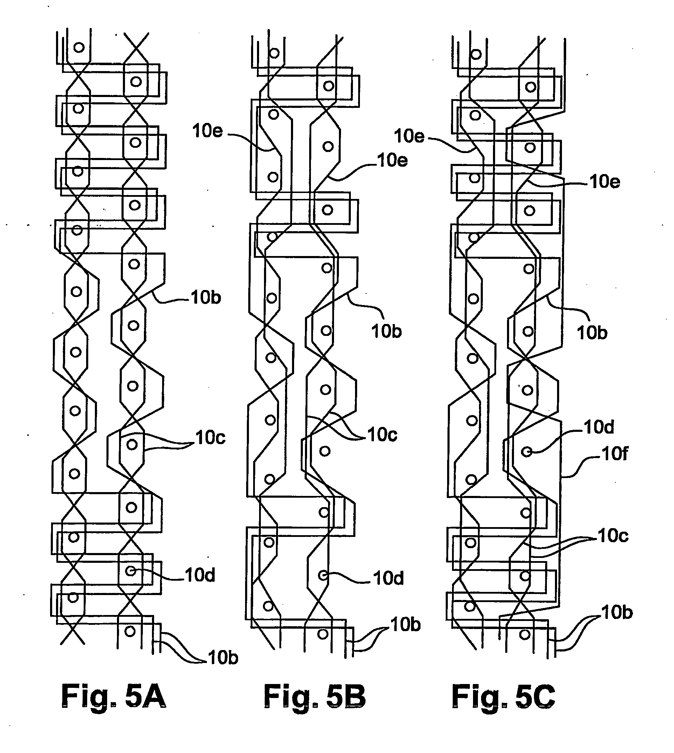

On a représenté, aux figures 5A, 5B, 5C, des exemples d'armure et de formation de poches non limitatifs. Pour l'identification des fils, les fils de chaîne sont identifiés par (10c), les fils de trame par (10d), les fils de liage par (11b), les fils élastiques par (10e) et d'éventuels fils d'envers par (10f).

Les avantages ressortent bien de l'invention. On souligne la nouvelle conception des tissus de contention par définition, lors de la fabrication, de poches réceptrices de moyens et composants extérieurs en réduisant de manière substantielle les coûts ultérieurs de confection. On souligne la diversité d'applications des tissus de contention et aussi leur possibilité d'adaptation aux besoins réels.

A titre indicatif, les tissus de contention à poches selon l'invention peuvent constituer des ceintures médicales pour le soutien lombaire, des ceintures post-opératoires ou abdominales, des orthèses, des atèles, des bandages ...

La mise en oeuvre de l'invention offre ainsi de très larges possibilités d'utilisation dans les meilleures conditions économiques.

En dernier lieu, les ouvertures préalables des poches peuvent rester libres ou autoriser l'enlèvement ou mise en place desdits moyens (11) à tout moment, ou être obturées par tous moyens, permettant une grande modularité ou versatilité.

## Revendications

1. Tissu de contention, du type agencé avec des logements de réception confectionnés et destinés à recevoir notamment des moyens de rigidification, **caractérisé en ce qu'il** comprend deux nappes tissées (A - B) solidaires par liage et s'écartant l'une de l'autre ponctuellement de manière non liée lors de leur fabrication pour définir directement une ou des poches (C) profilées et agencées pour recevoir un ou des moyens introduits après la fabrication, **et en ce que** la ou lesdites poches sont donc incorporées dans le corps du tissu de contention, chaque poche présentant une ouverture (10a) d'introduction d'un des moyens (11).

2. Tissu de contention, selon la revendication 1, **caractérisé en ce que** le ou lesdits moyens (11) introduits dans le tissu sont des raidisseurs du type baleines.

3. Tissu de contention, selon la revendication 1, **caractérisé en ce que** le ou lesdits moyens introduits dans le tissu ont une fonction thérapeutique et/ou de confort.

4. Procédé de confection de tissu de contention **caractérisé en ce qu'il** consiste à réaliser deux nappes tissées (A - B) qui sont superposées et solidaires par des fils de liage (10b) pour constituer des parties unitaires du tissu de contention, puis, selon un cycle pré-établi, à séparer lesdites nappes selon une distance donnée, puis à les rapprocher et solidariser à nouveau pour constituer successivement une ou des poches (C) profilées pour l'introduction et la réception ultérieure de moyens extérieurs (11),
**et en ce que,** simultanément à la fabrication du tissu de contention, la ou lesdites poches sont fermées partiellement en laissant une ouverture d'introduction (10a) pour la mise en place du ou desdits moyens précités.

5. Procédé de confection, selon la revendication 4, **caractérisé en ce que** les lisières sont établies, l'une étant fermée sur toute la longueur du tissu de contention, et l'autre étant fermée partiellement à l'exception de la zone des poches pour autoriser l'insertion desdits moyens dans lesdites poches.

6. Procédé de confection, selon la revendication 4, **caractérisé en ce que** les ouvertures (10a) des poches débouchent dans la largeur du tissu de contention.

7. Utilisation du tissu de contention, selon l'une quelconque des revendications 1 à 3, ou obtenu par la mise en oeuvre du procédé selon l'une quelconque des revendications 4 à 6, à la fabrication de ceintures médicales pour le soutien lombaire.

8. Utilisation du tissu de contention, selon l'une quelconque des revendications 1 à 3, ou obtenu par la mise en oeuvre du procédé selon l'une quelconque des revendications 4 à 6 à la fabrication de ceintures post-opératoires ou abdominales.

9. Utilisation du tissu de contention, selon l'une quelconque des revendications 1 à 3, ou obtenu par la mise en oeuvre du procédé selon l'une quelconque des revendications 4 à 6 à la fabrication d'orthèses.

10. Utilisation du tissu de contention, selon l'une quelconque des revendications 1 à 3, ou obtenu par la mise en oeuvre du procédé selon l'une quelconque des revendications 4 à 6 à la fabrication de bandages.

## Claims

1. Support fabric, of the type arranged with accommodating recesses prepared and intended in particular for accommodating stiffening means, **characterised in that** it comprises two woven plies (A - B) joined by bonding and separated from one another in places in an unbonded manner during their fabrication for directly defining one or more pockets (C) profiled and arranged to accommodate one or more means introduced after fabrication, **and in that** the said pocket or pockets are thereby incorporated in the body of the support fabric, each pocket having an opening (10a) for introducing one of the means (11).

2. Support fabric according to Claim 1, **characterised in that** the said means (11) introduced into the fabric are bone type stiffeners.

3. Support fabric according to Claim 1, **characterised in that** the said means introduced into the fabric have a therapeutic and/or comfort function.

4. Method for making support fabric, **characterised in that it** consists in preparing two woven plies (A - B) which are superimposed and joined by bonding yarns (10b) for constituting unitary parts of the support fabric, then, according to a predefined cycle, in separating the said plies along a given distance, then in bringing them together and joining them again to constitute a succession of one or more pockets (C) profiled for subsequently introducing and accommodating external means (II).

5. Method of preparation according to Claim 4, **characterised in that** the selvedges are established, one being closed along the whole length of the support fabric, and the other being partially closed with the exception of the area of the pockets, for inserting the said means into the said pockets.

6. Method of preparation according to Claim 4, **characterised in that** the openings (10a) of the pockets open onto the width of the support fabric.

7. Use of the support fabric according to any one of Claims 1 to 3, obtained by implementing the method according to any one of Claims 4 to 6, for fabricating medical belts for lumbar support.

8. Use of the support fabric according to any one of Claims 1 to 3, obtained by implementing the method according to any one of Claims 4 to 6, for fabricating post-operative or abdominal belts.

9. Use of the support fabric according to any one of Claims 1 to 3, obtained by implementing the method according to any one of Claims 4 to 6, for fabricating braces.

10. Use of the support fabric according to any one of Claims 1 to 3, obtained by implementing the method according to any one of Claims 4 to 6, for fabricating bandages.

## Patentansprüche

1. Stützgewebe, das mit Aufnahmen angeordnet ist, welche insbesondere Verstärkungselemente aufnehmen sollen, **dadurch gekennzeichnet, dass** es zwei Gewebelagen (A - B) aufweist, die durch Ketteln miteinander verbunden sind, und während ihrer Herstellung zeitweise einen Abstand zueinander aufweisen, um direkt eine profilierte Tasche oder mehrere profilierte Taschen (C) zu bilden, die ein Element oder mehrere Elemente aufnehmen sollen, welche nach der Herstellung eingeführt werden, und **dadurch gekennzeichnet, dass** die Tasche oder die Taschen somit in den Hauptteil des Stützgewebes integriert sind, wobei jede Tasche eine Öffnung (10a) zur Einführung eines der Elemente (11) aufweist.

2. Stützgewebe nach Anspruch 1, **dadurch gekennzeichnet, dass** es sich bei dem Element (11) oder bei den Elementen (11) um Versteifungen aus Stahl - oder Kunststoffstäbchen handelt.

3. Stützgewebe nach Anspruch 1, **dadurch gekennzeichnet, dass** das Element oder die Elemente, die in das Stützgewebe eingeführt werden, eine therapeutische Funktion und / oder eine Komfort - Funktion aufweisen.

4. Verfahren zur Herstellung des Stützgewebes, **dadurch gekennzeichnet, dass** es darin besteht, zwei Gewebelagen (A - B) herzustellen, die übereinander gelagert und durch Bindefäden oder Drehfäden (10b) miteinander verbunden sind, um einheitliche Teile des Stützgewebes zu bilden, und anschließend die Gewebelagen entsprechend einem vorher festgelegten Arbeitsablauf gemäß einem gegebenen Abstand zu teilen, um sie danach wieder aneinander anzunähern und erneut miteinander zu verbinden, so dass sie nacheinander eine profilierte Tasche oder profilierte Taschen (C) bilden, in die später externe Elemente (11) eingeführt und aufgenommen werden, und dass gleichzeitig mit der Herstellung des Stützgewebes die Tasche oder die Taschen teilweise geschlossen werden, wobei eine Einführungsöffnung (10a) für das Einsetzen des oben genannten Elementes oder die oben genannten Elemente verbleibt.

5. Herstellungsverfahren nach Anspruch 4, **dadurch gekennzeichnet, dass** die Ränder oder Säume hergestellt werden, indem einer über die gesamte Länge des Stützgewebes geschlossen wird, und der andere teilweise geschlossen wird, mit Ausnahme des Bereiches der Taschen, so dass die Elemente in die Taschen eingeführt werden können.

6. Herstellungsverfahren nach Anspruch 4, **dadurch gekennzeichnet, dass** die Öffnungen (10a) der Taschen in die Breite des Stützgewebes münden.

7. Verwendung des Stützgewebes nach einem der Ansprüche 1 bis 3 oder Herstellung durch das Verfahren nach einem der Ansprüche 4 bis 6 für die Produktion von medizinischen Gürteln zur Unterstützung der Lendenwirbel.

8. Verwendung des Stützgewebes nach einem der Ansprüche 1 bis 3 oder Herstellung durch das Verfahren nach einem der Ansprüche 4 bis 6 für die Produktion von postoperativen Gürteln oder Beckengürteln.

9. Verwendung des Stützgewebes nach einem der Ansprüche 1 bis 3 oder Herstellung durch das Verfahren nach einem der Ansprüche 4 bis 6 für die Produktion von Orthesen.

10. Verwendung des Stützgewebes nach einem der Ansprüche 1 bis 3 oder Herstellung durch das Verfahren nach einem der Ansprüche 4 bis 6 für die Produktion von Bandagen.
